# EUROPEAN PATENT SPECIFICATION

(11) **EP 4 282 921 B1**
(45) Date of publication and mention of the grant of the patent: **18.03.2026**
(21) Application number: 23174364.2
(22) Date of filing: 19.05.2023
(51) Int. Cl.: C08L 89/00, A61K 31/05, A61L 15/22, A61P 43/00, C07K 14/425, C08L 39/06

(54) **BIOCOMPATIBLE AND BIODEGRADABLE COMPOSITE MATERIAL FOR UNDERWATER DELIVERY ACTIVE AGENTS AND USES THEREOF**
BIOKOMPATIBLES UND BIOLOGISCH ABBAUBARES VERBUNDMATERIAL FÜR DIE ABGABE VON WIRKSTOFFEN UNTER WASSER UND IHRE VERWENDUNG
MATÉRIAU COMPOSITE BIOCOMPATIBLE ET BIODÉGRADABLE POUR L'ADMINISTRATION DES AGENTS ACTIFS SOUS-MARINS ET UTILISATIONS DE CEUX-CI

(30) Priority: 25.05.2022 IT 202200010895
(43) Date of publication of application: 29.11.2023
(73) Proprietor: Fondazione Istituto Italiano di Tecnologia, 16163 Genova (IT); Università degli Studi di Milano - Bicocca, 20126 Milano (IT)
(72) Inventor: CONTARDI, Marco, 90147 Palermo (IT); MONTANO, Simone, 20037 Paderno Dugnano (MI) (IT); GALLI, Paolo, 20147 Milano (IT); ATHANASIOU, Athanasia, 16014 Ceranesi (GE) (IT)
(74) Representative: Buzzi, Notaro & Antonielli d'Oulx S.p.A.

(56) References cited:
- WO-A1-2018/039739
- CN-A- 111 729 122
- CONTARDI MARCO ET AL: "Treatment of Coral Wounds by Combining an Antiseptic Bilayer Film and an Injectable Antioxidant Biopolymer", vol. 10, no. 1, 22 January 2020 (2020-01-22), XP093007024, Retrieved from the Internet <URL:https://www.nature.com/articles/s41598-020-57980-1> DOI: 10.1038/s41598-020-57980-1
- DAVID J. SESSA ET AL: "Melt-processed blends of zein with polyvinylpyrrolidone", INDUSTRIAL CROPS AND PRODUCTS, vol. 33, no. 1, 1 January 2011 (2011-01-01), NL, pages 57 - 62, XP055658275, ISSN: 0926-6690, DOI: 10.1016/j.indcrop.2010.08.008
- LESSER M P: "Oxidative stress causes coral bleaching during exposure to elevated temperatures", CORAL REEFS ; JOURNAL OF THE INTERNATIONAL SOCIETY FOR REEF STUDIES, SPRINGER, BERLIN, DE, vol. 16, no. 3, 20 October 1997 (1997-10-20), pages 187 - 192, XP035128586, ISSN: 1432-0975, DOI: 10.1007/S003380050073

## Description

### Field of the invention

This disclosure generally relates to a biocompatible and biodegradable composite material for the underwater release of active agents. More particularly, the disclosure refers to a composite material biocompatible and biodegradable in an aquatic marine environment for the underwater release of at least one active agent useful for preventing and/or treating coral bleaching.

### Background

Coral reefs represent a unique ecosystem, characterized by a high degree of biodiversity.

This incredible ecosystem - capable of supporting the lives of millions of people around the world - is running up against significant suffering due mostly to climate and environmental changes caused by human activities. The pollution generated by the use of plastic, the intensive fishing, the reduction of pH, the increase of pathologies and temperature of the sea surface are negatively affecting the biological cycle and the survival of coral reefs.

Corals are animals, in particular colonies of polyps that live in symbiosis with unicellular algae commonly known as zooxanthellae, the latter belonging to the Symbiodiniaceae family. The polyps obtain most of the energy for their survival from the products of the photosynthesis of the algae and, to a lesser extent, by taking plankton and small organisms.

Most corals live at a temperature between 25°C and 28°C and even minimal changes in this climatic condition are able to exert significant stress on them. In particular, it has been demonstrated that conditions of thermal stress, both from cold and heat, can lead to the death of corals. Conditions of thermal stress can be determined by temporary or permanent changes in the temperature of the sea water due to the alternation of the seasons, the construction of man-made infrastructures, the global climate change.

In particular, the increase in sea surface temperature can lead to coral bleaching as it is able to alter the symbiotic relationship between the polyp and the algae. As a consequence, the loss of contact with the algae and their photosynthetic pigments makes the polyps transparent, so much that their white calcium carbonate skeleton can be seen.

In particular, the potentially underlying phenomenon of coral bleaching is to be found in an increase in oxidative stress on the algae. Scientific literature has shown that corals are able to activate bio-molecular antioxidant pathways, inducing the expression of proteins such as, for example, the enzymes superoxide dismutase (SOD), catalase and glutathione peroxidase (GPX), the tripeptide glutathione (GSH), other heat shock proteins.

Similar protein activations are also found in humans and other animals and the administration of antioxidant compounds is often a useful strategy in order to counteract oxidative damage. Natural antioxidants, such as for example curcumin, polyphenols, phenolic acids, anthocyanins, flavonoids, beta-carotene can be used for this purpose. For higher organisms, such as humans and domestic animals, specific transport systems have been developed capable of selectively releasing the active compounds to the target of interest. Such transport and release systems - for a biomedical application aimed at the human body - can include, for example, films, hydrogels, particles, fibers.

With reference to corals, the implementation of a strategy capable of counteracting oxidative stress and consequently their bleaching encounters, however, operational difficulties due to the need to release antioxidant compounds into the aquatic environment.

The document Contardi et al. Treatment of Coral Wounds by Combining an Antiseptic Bilayer Film and an Injectable Antioxidant Biopolymer. Sci Rep 10, 988 (2020), for example, discloses a two-step method for the release of antibiotics (anti-fungal or anti-protozoal) comprising i) a first step in which a hydrophilic film containing an antiseptic agent is wrapped on the corals to be treated and ii) a second step in which a composition in the form of a paste containing coumaric acid and caprolactone is contacted with the portion of coral to be treated.

Other technical solutions currently known include, for example, the pulsed administration of cold water from the depth of the sea in order to reduce the increase in surface temperature or "shading" techniques, with which to reduce the light exposure of the corals.

### Summary of the invention

This disclosure aims to provide a composite material for the controlled underwater release of active agents, particularly effective for preventing and/or treating coral bleaching.

According to this disclosure, the above object is achieved thanks to the
subject-matter to which specific reference is made in the following claims, intended as an integral part of this disclosure.

The references to methods of treatment in the detailed description of the invention are to be interpreted as references to the compounds, pharmaceutical compositions and medicaments of the present invention for use in a method for treatment of the human (or animal) body by therapy.

The invention is set out in the appended set of claims.

This disclosure provides a composite material biocompatible and biodegradable in an aquatic environment, preferably in a marine environment, for the underwater release of at least one active agent, wherein the composite material comprises at least one hydrophobic compound, at least one hydrophilic compound, at least one active agent.

The at least one hydrophobic compound may be a polymer selected from the group consisting of poly(butyladipate-co-terephthalate) (PBAT), polyhydroxyalkanoates (PHA), zein, silk proteins, albumin, keratin, casein, gelatin, thermoplastic starch.

The at least one hydrophilic compound may be a polymer selected from the group consisting of polyvinylpyrrolidone (PVP), polyvinyl alcohol (PVA), polyethylene glycol (PEG), polyethylene oxide (PEO), a polysaccharide, preferably selected from alginate, chitosan, pectin, agar, hyaluronic acid.

The active agent may comprise at least one antioxidant compound, preferably selected from curcumin, polyphenols, phenolic acids, anthocyanins, flavonoids, beta-carotene, lignin, ascorbic acid, vitamins. Further usable antioxidant compounds are butylhydroxyanisole and butylhydroxytoluene.

In a preferred embodiment according to the invention, the composite material comprises zein as the at least one hydrophobic compound, polyvinylpyrrolidone (PVP) as the at least one hydrophilic compound, curcumin as the at least one active agent.

In one or more embodiments, the composite material may further comprise at least one plasticizer, such as for example glycerol.

In one or more embodiments, the composite material may further comprise at least one emulsifier, preferably selected from soy lecithin, cyclodextrins, natural rubber such as for example Astragalus tragacanth gum (Astragalus gummifer gum). The emulsifier may promote the dispersion of the active agent, preferably of the antioxidant compound, in the composite material.

In one or more embodiments, the composite material is in the form of a film. The film may have a thickness ranging from 20 µm to 1.5 cm, preferably from 150 µm to 350 µm.

The disclosure further provides a method for preparing the biocompatible and biodegradable composite material comprising the step of dissolving the at least one hydrophilic compound, the at least one hydrophobic compound, the at least one active agent, and optionally at least one of a plasticizer and an emulsifier, in a solvent to form a solution. In one or more embodiments, the method may further comprise the step of distributing the solution obtained on the surface of a support and evaporating the solvent to obtain the biocompatible and biodegradable composite material in the form of a film.

The composite material, subject of this disclosure, can be used for the underwater release of an active agent suitable for counteracting coral bleaching. Preferably, the composite material may be placed underwater in contact with at least one portion of the surface of at least one coral to be treated, preferably it may be wrapped around at least one portion of the surface of the coral. In one or more embodiments, the composite material may be placed underwater in proximity of the surface of at least one coral to be treated, preferably attached to a support or coated to a support. The support may be selected, for example, from nettings, wires, tubes, coral nurseries.

In one or more embodiments, the disclosure provides a method for the underwater release of at least one active agent suitable for preventing and/or treating coral bleaching, which comprises the step of immersing the biocompatible and biodegradable material, subject of this disclosure, in an aquatic environment. Preferably, the method may comprise the step of:
- contacting the biocompatible and biodegradable composite material with at least one portion of the surface of at least one coral, more preferably wrapping the biodegradable composite material in the form of a film around at least one portion of the surface of the coral, and/or
- placing the biodegradable composite material in proximity of at least one portion of the surface of at least one coral, wherein more preferably the biodegradable composite material adheres to a support, preferably selected from wires, nettings, tubes, coral nurseries.

In one or more embodiments, the disclosure further provides a method for preventing and/or treating coral bleaching, comprising the step of: underwater contacting the biodegradable composite material with at least one portion of the surface of at least one coral, preferably wrapping said biodegradable composite material in the form of a film around at least one portion of the surface of the coral. In one or more embodiments, the method for preventing and/or treating coral bleaching may comprise the step of underwater placing the biodegradable composite material in proximity of at least one portion of the surface of at least one coral, wherein preferably said biodegradable composite material is in the form of a film and, more preferably, adheres to a support, the support being preferably selected from wires, nettings, tubes, coral nurseries.

The disclosure further provides a kit for producing the biocompatible and biodegradable composite material comprising at least one hydrophobic compound, at least one hydrophilic compound, optionally at least one active agent, optionally at least one plasticizer and/or one emulsifier.

The composite material is a composite material biocompatible and biodegradable in the marine aquatic environment and may therefore be released into the environment without pollution problems; moreover, it does not have any effect on the vitality of aquatic species, such as corals for example. The composite material may also release the active agent contained therein in a controlled manner, i.e. slowly or rapidly, depending, for example, on the time duration of the temperature fluctuation. The release of antioxidant compounds, for example curcumin as active agents, has also proved to be particularly effective in counteracting the phenomenon of coral bleaching.

### Brief description of the drawings

The invention will now be described, only by way of example, with reference to the attached figures, in which:

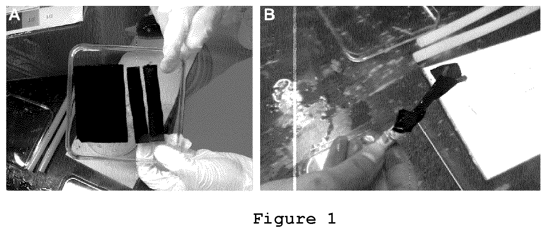

- Figure 1, panel A, shows a photo of an example of composite material in the form of a film, separated into strips; the composite material comprises the hydrophobic compound, zein, and the hydrophilic compound, PVP (indicated by P in the figures), at a weight ratio of 6/4 (6 parts by weight of zein and 4 parts by weight of PVP) and curcumin. Panel B shows a photo of a coral on which a strip of the film illustrated in panel A has been wrapped and attached to a support;

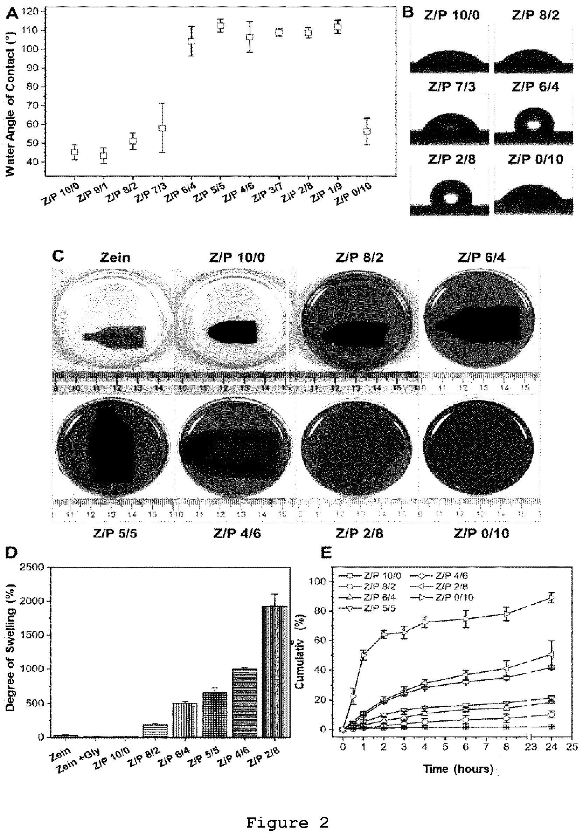

- Figure 2, panel A, shows the results of an analysis of the contact angle with water of composite material wherein the weight ratio of zein to PVP is equal to 10/0, 9/1, 8/2, 7/3, 6/4, 5/5, 4/6, 3/7, 2/8, 1/9, 0/10. Panel B shows photos of the contact angle, after 15 seconds, of the samples with a weight ratio of zein to PVP equal to 10/0, 8/2, 7/3, 6/4, 2/8, 0/10. Panel C shows photos of examples of composite material including pure zein (without curcumin) and samples with curcumin, wherein the weight ratio of zein to PVP is equal to 10/0, 8/2, 6/4, 5/5, 4/6, 2/8, 0/10 after 24 hours of immersion in salt water. Panel D represents a graph showing the percentage swelling values for samples with pure zein (without curcumin), pure zein with glycerol (without curcumin), and for samples with curcumin and ratios of PVP to zein equal to 10/0, 8/2, 6/4, 5/5, 4/6, 2/8. In panel E, the graph shows curcumin release profiles at 25°C from films with a ratio of zein to PVP equal to 10/0, 8/2, 6/4, 5/5, 4/6, 2/8, 0/10;

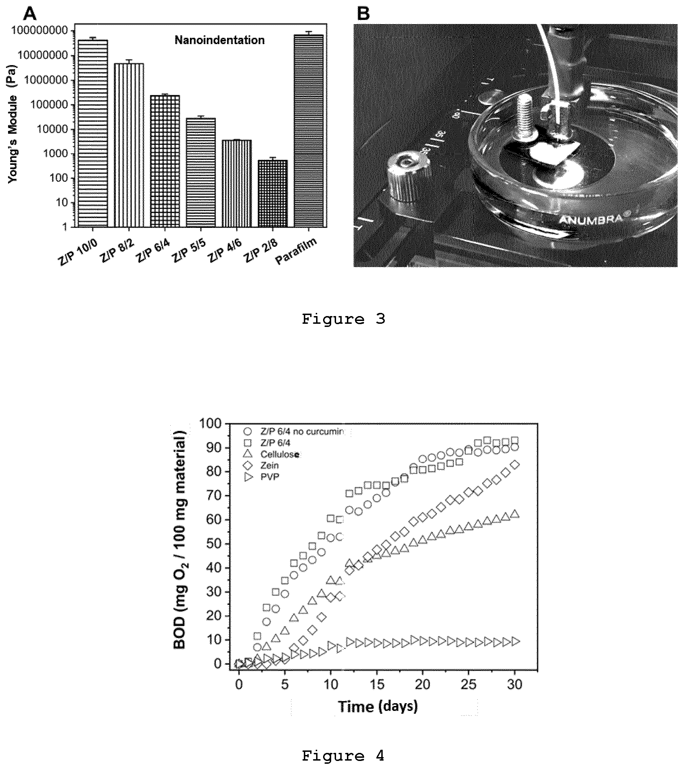

- Figure 3, panel A, shows a graph indicating the values of the Young's modulus obtained by nanoindentation and after 24 hours of immersion in salt water of the samples, in the form of a film, with curcumin and with a ratio of zein to PVP equal to 10/0, 8/2, 5/5, 4/6, 2/8, and parafilm (that is, a film of polyolefins and paraffin wax); panel B shows a photo of a composite material in the form of a film comprising curcumin, and PVP and zein at a weight ratio of 6/4, immersed in salt water in the set-up for the nanoindentation;
- Figure 4 is a graph of the biochemical oxygen consumption (expressed as mg of O₂/100 grams of material) as a function of time (in days) for composite material samples comprising only PVP without curcumin, only zein without curcumin, zein and PVP at a weight ratio of 6/4 without curcumin, zein and PVP at a weight ratio of 6/4 with curcumin, cellulose;

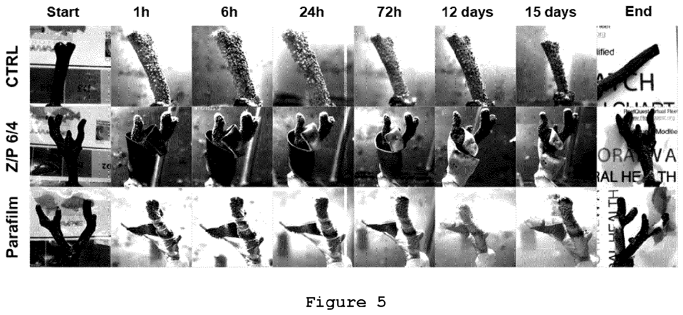

- Figure 5 shows photos of untreated corals (CTR), corals treated with a composite material comprising zein and PVP at a weight ratio of 6/4 with curcumin, corals treated with parafilm at different time intervals within 15 days;

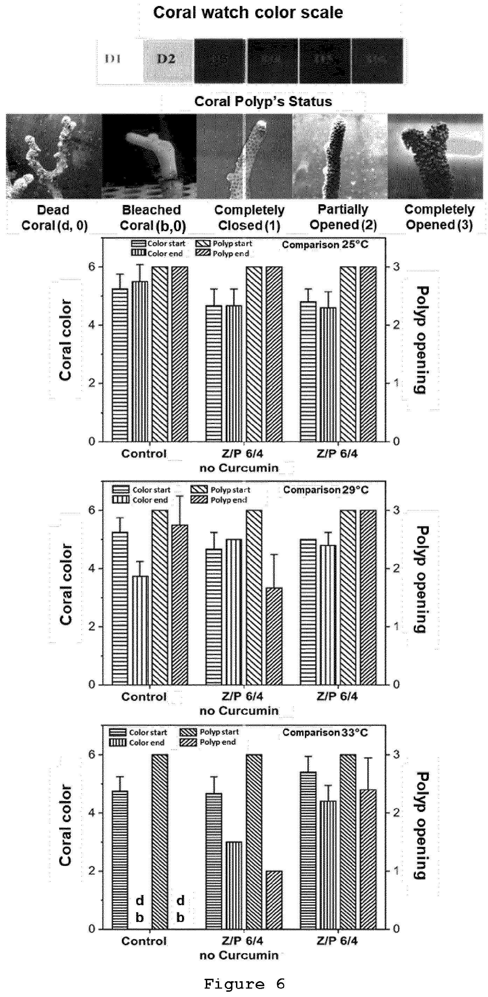

- Figure 6 shows from top to bottom the reference color chart for the color of the corals, photos of different conditions of polyp opening, monitoring of the condition of untreated corals, corals treated with samples of material in the form of a film with a ratio of zein to PVP of 6/4 without curcumin, and with samples of material in the form of a film with a ratio of zein to PVP of 6/4 with curcumin, at temperatures of 25°C, 29°C and 33°C before and after 36 hours;

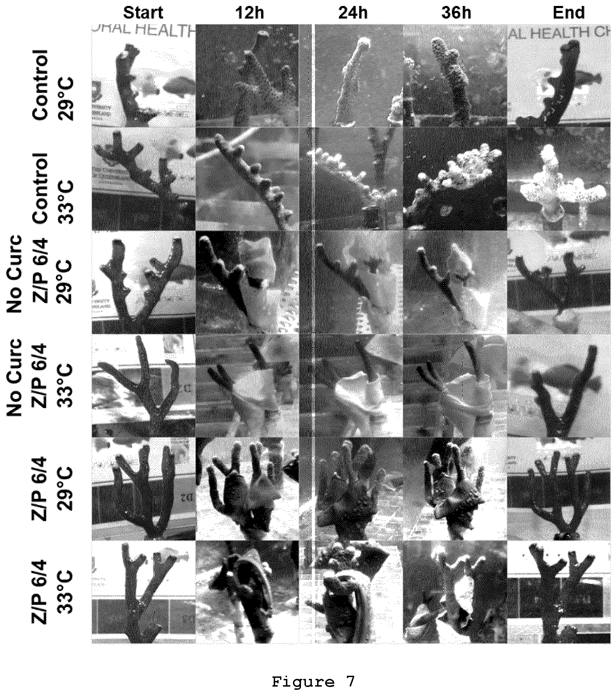

- Figure 7 shows photos of untreated corals, corals treated with a material comprising zein and PVP at a weight ratio of 6/4 without curcumin and a material comprising zein and PVP at a weight ratio of 6/4 with curcumin at 29°C and 33°C at different time intervals for 36 hours.

### Detailed description of preferred embodiments

This disclosure provides a biocompatible and biodegradable composite material for the underwater release of active agents, wherein the composite material comprises at least one hydrophobic compound, at least one hydrophilic compound, at least one active agent.

The hydrophobic compound ensures the underwater resistance of the material while the hydrophilic compound allows water to enter the material, aiding both the release of the active agent and the biodegradation process of the same material.

The at least one hydrophobic compound may be selected from the group consisting of poly(butyladipate-co-terephthalate) (PBAT), polyhydroxyalkanoates (PHA), zein, silk proteins, albumin, keratin, casein, gelatin, thermoplastic starch.

The at least one hydrophilic compound may be selected from the group consisting of polyvinylpyrrolidone (PVP), polyvinyl alcohol (PVA), polyethylene glycol (PEG), polyethylene oxide (PEO), at least one polysaccharide, preferably selected from alginate, chitosan, pectin, agar, hyaluronic acid.

The term "composite material" used in this disclosure means a product, a composition which includes two or more components with different features.

The term "active agent" means any substance with a specific biological activity, of natural, synthetic or semi-synthetic origin.

The composite material is a biodegradable composite material. The term "biodegradable" means a material which, dispersed in the environment, decomposes thanks to the action of bacteria or other microorganisms, into substances such as carbon dioxide (CO₂) and water (H₂O).

In one or more embodiments of the composite material subject of this disclosure, the active agent may comprise at least one antioxidant compound of natural origin, preferably selected from curcumin, polyphenols, phenolic acids, anthocyanins, flavonoids, beta-carotene, lignin, ascorbic acid, vitamins. Further usable antioxidant compounds are butylhydroxyanisole and butylhydroxytoluene.

Unlike active agents, such as antibiotics, anti-fungal or anti-protozoal agents, the use of antioxidant compounds of natural origin has the advantage of not making it necessary to extract the corals from the water in order to be treated; this is because the antioxidant agent of natural origin may be released into the water without negative effects on the surrounding environment.

The active agent may be contained in the composite material at an amount by weight ranging from 1% to 35%, preferably ranging from 1% to 10% by weight relative to the total weight of the hydrophobic compound and the hydrophilic compound.

In one or more embodiments, the composite material may further comprise at least one plasticizer, such as for example glycerol. The plasticizer aids the conferring of specific mechanical properties to the material, such as breaking resistance and flexibility. The plasticizer may be contained in the composite material at an amount by weight ranging from 0% to 35% relative to the total weight of the hydrophobic compound and the hydrophilic compound.

In one or more embodiments, the composite material may further comprise at least one emulsifier, for example soy lecithin. The emulsifier may be present at an amount ranging from 0% to 35% by weight relative to the total weight of the hydrophobic compound and the hydrophilic compound.

The composite material may comprise a hydrophobic compound and a hydrophilic compound at a weight ratio ranging from 9/1 to 2/8. By the designation 9/1 and 2/8 are meant 9 parts by weight of hydrophobic compound and 1 part by weight of hydrophilic compound as well as 2 parts by weight of hydrophobic compound and 8 parts by weight of hydrophilic compound, respectively.

Preferably, the hydrophobic compound is present in the composite material at an amount equal to or greater than 20% by weight relative to the total weight of the hydrophobic compound and the hydrophilic compound.

The weight ratio may be changed depending on the features of the active agent contained in the composite material, in particular depending on the active agent partition coefficient expressed as logP of the concentration ratio of the active agent in oil and in water.

This weight ratio may also change as a function of any hydrogen (H) type interactions, hydrophobic interactions (for example Van der Waals forces), electrostatic interactions with the polymers used. Furthermore, it may also depend on the more or less crystalline and/or amorphous nature of the agent used, which may be a limitation in its dispersion within the matrix and which therefore involves an adjustment of the formulation.

To aid a controlled (not immediate) release of highly water-soluble active agents, the weight ratio of the hydrophobic compound to the hydrophilic compound may be biased in favor of a higher amount of hydrophobic compound compared to the hydrophilic compound. Conversely, in the case of a hydrophobic active agent, such as curcumin, the composite material may comprise a greater amount of hydrophilic compound than that of the hydrophobic compound.

The presence of at least one plasticizer, for example glycerol, allows to obtain optimal mechanical properties of the composite material with regard to flexibility and breaking resistance.

In one or more embodiments, the composite material may be in the form of films, hydrogels, spheres, fibers (microfibers or nanofibers), extruded filaments, combinations thereof, for example fibers or particles dispersed in a single layer or double layer film. For this purpose, the specific shape of the material may be obtained by, for example, 3D molding, injection-molding, hot-press, spin-coating, rod-coating, electrospinning.

The composite material in the form of a film may have a thickness ranging from 20 µm to 1.5 cm, preferably from 150 µm to 350 µm.

Advantageously, the composite material in the form of a film may be wrapped around the surface of a coral.

In a preferred embodiment according to the invention, the composite material comprises zein as the at least one hydrophobic compound, polyvinylpyrrolidone (PVP) as the at least one hydrophilic compound, curcumin as the at least one active agent.

Zein is a prolamine contained in corn which may be obtained as a by-product of its processing. It is an edible protein, water-insoluble, used in various applications and industrial fields, from biomedical to food packaging. The presence of zein in the composite material, subject of this disclosure, has proved to be particularly suitable for encapsulating hydrophobic molecules such as, for example, curcumin.

PVP is a hydrophilic polymer generally used in the production of pharmaceutical and cosmetic products. The presence of PVP favors the penetration of water inside the composite material and the release of the active agent, such as curcumin.

Curcumin is a natural antioxidant molecule found for example in curry and widely used in Indian and Asian cultures. It is a molecule that is poorly water-soluble and has antioxidant properties.

In one or more embodiments, the composite material may comprise zein and PVP at a weight ratio ranging from 9/1 to 2/8, preferably equal to 6/4, and curcumin at an amount by weight ranging from 1% to 35%, preferably ranging from 1% to 35% relative to the total weight of the hydrophobic compound and the hydrophilic compound.

The composite material may be obtained by dissolving the hydrophilic compound, the hydrophobic compound and the active agent, and optionally at least one plasticizer and/or one emulsifier, in a solvent to form a solution. In one or more embodiments, the solvent may be selected from the group consisting of chloroform, methanol, DMSO, ethanol, water and combinations thereof. The solution may be kept under stirring for a period ranging from 1 hour to 30 days, preferably for 24 hours. The solution may be maintained at a temperature between 5°C and 120°C. The temperature may be changed depending on the solvent used, in particular depending on the boiling temperature of the same.

In one or more embodiments, to obtain the composite material in the form of a film, the solution may be distributed (applied) on a flat surface of a support, for example Petri dishes, for example made of glass, plastic or Teflon depending on the solvent used. After the distribution of the solution on the support, for example by casting, a phase of evaporation of the solvent follows. In one or more embodiments, the evaporation of the solvent may be carried out by heating the solution to a temperature ranging from 35°C to 120°C, preferably equal to 60°C. The phase of heating the solution may be carried out for a time period ranging from 1 hour to 7 days, preferably for 2 days, more preferably in dark conditions. The film thus obtained may be detached from the support and optionally separated into strips or any other desired shape for its use.

The composite material, subject of this disclosure, may be used in a method for the underwater release of at least one active agent, wherein the method comprises the step of placing the composite material in water for the release of the active agent. As demonstrated below, the composite material, subject of this disclosure, has proved to be particularly effective for preventing and treating coral bleaching. In particular, the experimental tests demonstrate the effectiveness of the material in the form of a film contacted with the surface of corals subjected to thermal stress, in particular wrapped to coat at least one portion of the coral surface.

In one or more embodiments, the composite material may also be attached on various types of support, such as for example nettings, wires, tubes, coral nurseries to be immersed both in aquarium tanks and in a marine environment. Preferably, the support coated with the composite material may be placed in proximity of a coral colony, in particular at a minimum distance from the colony, such as to allow effective release of the active agent towards the target. The composite material, preferably in the form of a film, may for example coat (as a coating) supports generally used in an aquatic environment (in aquarium tanks or in a marine environment). For this purpose, the composite material in the form of a solution comprising the hydrophilic compound, the hydrophobic compound, the active agent, optionally the plasticizer and/or the emulsifier, may be distributed, for example by brushing or spraying, on the chosen support (for example nursery, PVC tubes, nettings, wires). Alternatively, the support may be immersed (dip coating) in this solution. The support is consequently dried, for example with hot air generators, in order to obtain the evaporation of the solvent and the solidification of the solution to obtain a film.

The biodegradation of the material and the progressive release of the antioxidant agent are able, in this way, to counteract the phenomenon of coral bleaching.

### EXAMPLES

### Example 1. Preparation of the composite material in the form of a film and use thereof

Composite materials in the form of a film were prepared by dissolving, in a solvent comprising DMSO and methanol (1:3; 6 mL DMSO, 18 mL methanol), zein, PVP, glycerol, and curcumin. The final concentration of the two polymers (zein and PVP) relative to the final volume of the solvent was kept equal to 7% weight/volume (w/v). Glycerol was added at a concentration equal to 10% w/w relative to the total weight of the two polymers (zein and PVP).

Curcumin was used at a concentration of 2.4% w/w relative to the total weight of the two polymers (zein and PVP).

Composite materials were also prepared in the form of films in which the zein/PVP weight ratio ranged from 10/0 to 0/10.

In particular, zein powder was first dissolved in 6 mL of DMSO and stirred for 6 hours at room temperature. Meanwhile, a solution of methanol with glycerol was prepared and added to the zein solution. Finally, PVP and curcumin were added to the solution and stirred for 24 hours to form a solution. Subsequently, the solution was poured into square Teflon molds of dimensions equal to 9 × 9 cm placed on a plate heated to 60°C for 2 days, in the dark, under a fume hood (in which the environmental conditions were adjusted at a temperature between 16°C and 20°C and a percentage of relative humidity (RH) between 40% and 50%).

Different weight ratios of the hydrophilic compound to the hydrophobic compound were tested in order to identify ratios that would allow for a composite material strength in water or a rapid dissolution. The inventors have observed that by increasing the concentration of the hydrophobic compound with respect to the hydrophilic one, a decrease in the dissolution rate is obtained. The ratio of the two compounds (for example zein and PVP) may be selected depending on the need to release the active agents quickly or slowly in aquatic environments, such as for example sea, lakes, rivers, aquariums.

From the films obtained as described above - which had a thickness ranging from 150 µm to 350 µm - strips of a rectangular shape were cut out (dimensions of 1.5 × 9 cm) which were contacted with coral fragments in order to obtain a localized release of the active agent. Figure 1A shows a photo of a strip of composite material (with zein/PVP weight ratio equal to 6/4) attached to a coral fragment anchored to a support. Attaching on the support was obtained with hot silicone glue by attaching the film between the coral support and the coral itself (Figure 1B).

In particular, the composite material was attached with hot glue to a plug used as a support for the cutting (a small fragment of coral).

The adhesive strength of the PVP initially played an important role in attaching the material to the coral, thanks to the adhesive properties of the PVP in the presence of water remaining superficially on the coral. Then, during the dipping, the composite swells and becomes a soft hydrogel which delicately wraps the coral without causing undesirable effects, as it does not block it and allows the exchange of oxygen and nutrients with the external environment (which instead does not allow the parafilm which, on the contrary, blocks it).

In order to understand the effect of global and local stressors on coral reefs, it is important to investigate the resilience of key structure-forming organisms, such as hard corals. Sometimes called the "lab mouse" of coral research, *Stylophora pistillata* (Esper, 1797) is one of the world's most studied nominal species of coral, which has been used extensively in stress experiments. *Stylophora pistillata* is a zooxanthellate scleractinian coral belonging to the Pocilloporidae family. It is a hardy branching hermatypic species with a branching growth form characterized by various colors from dark brown, purple and yellow to pale pink. Its colony morphology is highly plastic, leading to different architectures dynamically shaped by the environment. It is also considered an excellent model for the study of r-strategy (as it, for example, exhibits rapid growth, high population turnover) and has been widely used as a model system in studies of cnidarian physiology, reproduction, phenotypic plasticity, molecular biology.

### Example 2. Evaluation of the interaction of the composite material with water

The behavior and interaction of the composite material, subject of this disclosure, in the form of a film was evaluated by i) analysis of the contact angle with water (WCA), ii) swelling and iii) release properties of the active agent in water. This allowed testing of the composite material and coral response under simulated warming stress underwater over a 36-hour period.

The results and images of the WCA measurements are shown in Figures 2A and 2B, respectively. The inventors observed different values of the contact angle with water as a function of the weight ratio of the amount of zein to the amount of PVP. In particular, the samples with a high concentration of zein showed a value of about 50° (samples in which the weight ratio of zein to PVP was equal to 10/0, 9/1, 8/2, 7/3). The value shows an increase, up to 110°, with decreasing amount of zein (samples with zein/PVP weight ratio equal to 6/4, 5/5, 4/6, 3/7, 2/8, 1/9). The trend is reversed in the samples in which the zein/PVP weight ratio is 0/10.

The swelling properties of the composite material were evaluated following immersion of the films in sea water for 24 hours. The photos and analysis of the results are shown in Figures 2C and 2D, respectively. Samples in which the material included zein alone, or zein and glycerol, or zein and PVP at a ratio of 10/0 showed a swelling degree (SD) ranging from 10% to 20%, suggesting that curcumin did not directly affect this property.

In the samples in which the material included increasing amounts of PVP with respect to zein, the swelling degree of the material showed increasing values. For example, the sample where the material included zein and PVP at a ratio of 8/2 showed a swelling value of approximately (≈) 185%, while in the samples with a zein/PVP ratio of 6/4, 5/5, 4/6, 2/8 the swelling of the material reached values equal to ≈ 500%, 660%, 1000%, 1925%, respectively. Material samples with a zein/PVP ratio of 1/9 and 0/10 were completely dissolved within 24 hours, and therefore no swelling value could be calculated.

The results obtained from the analysis of the curcumin release profile from composite materials with different zein/PVP weight ratios are shown in Figure 2E. As can be seen, the fastest release of active agent is observed in samples of material with a zein/PVP ratio of 0/10, with approximately 75% of the amount of curcumin released in the first 4 hours.

The presence of zein in the composite material slows down the release of the active agent; this is due to the hydrophobic behavior of zein and the strong interaction with the poorly water-soluble antioxidant. Samples of material comprising zein and PVC at a ratio of 2/8 and 4/6 released a total amount of curcumin after 24 hours of about 50% and 40%, respectively. Further increasing the amount of zein within the material results in a slow and controlled release profile of curcumin, with less than 20% release after 24 hours. This condition can be considered suitable for a sustained release of active agents.

### Example 3. Mechanical properties

The mechanical properties of composite material samples were analyzed using the nanoindentation technique after immersion in salt water under films for 24 hours. A strip of parafilm was used as an experimental test control as physical obstacles such as sediments, sand or plastic pollution can affect the health of the corals.

Figures 3A and 3B show the results of the analysis in which it is evident that the parafilm sample showed the highest value of the Young's Modulus (MY), of about 66±27 MPa. The material with a zein/PVP ratio of 10/0 showed a behavior similar to that of parafilm, with a MY value of about 43±10 MPa. The presence of PVP in the composite material causes a drastic reduction of the MY value. The sample with a Z/P ratio of 6/4 had a MY value of about 0.24±0.036 MPa, while for the sample with a Z/P ratio of 2/8 the value was about 526±170 Pa.

The results described consequently suggest that the mechanical properties of the disclosed composite material can be modulated by the change of the zein/PVP weight ratio. The material can therefore be rigid and not swollen (for example with Z/P ratios of 10/0) or in the form of a very ductile, soft and swollen hydrogel (for example in the case of materials with a Z/P weight ratio of 2/ 8).

### Example 4. Biodegradability

The disclosed composite material is completely biodegradable and biocompatible, i.e. it does not have any type of polluting impact on the marine ecosystem.

The inventors conducted experimental tests in order to check the biochemical oxygen demand (BOD) of material samples in the form of films comprising PVP only, zein only, zein and PVP at the weight ratio of 6/4 without curcumin, zein and PVP at the weight ratio of 6/4 with curcumin, over a period of 30 days. Cellulose films were tested for the same time period as biodegradation positive control.

The results obtained are reported in Figure 4 where it can be noted that the PVP film showed the slowest degradation rate, obtaining a final value of 9 mg of O₂/100 mg of material. On the other hand, cellulose produced 63 mg of O₂/100 mg of material while films made of zein only, films including zein and PVP at a weight ratio of 6/4 without curcumin and with curcumin showed values of 83, 90 and 93 mg of O₂/100 mg of material, respectively, proving to be even more degradable than cellulose.

Samples comprising zein and PVP at a weight ratio of 6/4 started their degradation immediately, even earlier than films made of zein only.

This difference in degradation compared to pure compounds can be justified by the synergy between zein, a protein easily attacked by bacteria, and the high hydrophilicity of PVP, which induces swelling of the material, allowing water, and consequently bacteria, to entry into the bulk of the material. This observation is even more evident in the first days of the test, where both the samples with zein and PVP at a ratio of 6/4 without curcumin and the samples with zein and PVP at a ratio of 6/4 with curcumin started their degradation after only 2 days while pure zein films started their degradation after 6 days.

### Example 5. Biocompatibility with corals

Samples of composite material comprising zein and PVP at a ratio of 6/4 in the form of films were applied directly in contact with the corals, as shown in Figure 1B. Naked control corals and parafilm coated corals were also evaluated. Parafilm was chosen as a control to simulate a plastic material that can get stuck in the branches of corals.

The corals were placed in a tank at 25°C for 15 days. The color chart assessed the initial health status of the corals. Subsequently, images of the corals were acquired at different time intervals, and the main results are reported in Figure 5.

As can be seen, both naked and untreated control corals and film-coated corals comprising zein and PVP did not show any significant morphological and color changes.

On the other hand, after 15 days, the parafilm-wrapped coral showed obvious bleaching and stress phenomena. This confirms that both the composition of the material and the mechanical properties (a soft hydrogel of the biocomposite) did not influence the vitality of the corals demonstrating their total biocompatibility.

### Example 6. Thermal stress test

Coral bleaching episodes are mostly associated with temporary or permanent seawater warming events.

In order to simulate the warming stress condition and to test the effectiveness of the material subject of this disclosure, the corals were placed in tanks at 25°C, 29°C and 33°C for 36 hours.

Naked untreated control corals and corals treated with materials in the form of a film comprising zein and PVP at a weight ratio of 6/4 with and without curcumin were tested.

Two main parameters were monitored to evaluate the difference between the different stress conditions and treatments: the color change and the state of the polyp. The main observation results and the scale of monitoring conditions are shown in Figure 6. The photos of the experiments at 29°C and 33°C are shown in Figure 7.

At 25°C no evident changes were observed compared to the initial condition. This confirms the excellent biocompatibility of the composite material applied in direct contact with the corals.

At 29°C, on the other hand, the first symptoms of warming stress were noted. Indeed, a loss of color and a slight reduction in the polyp opening were recorded for the control samples.

A reduction in the polyp opening was also noted for the corals tested with the composite material without curcumin, but no evident color change was observed.

Corals wrapped with the composite material with and without curcumin showed no signs of stress after 36 hours at 29°C.

At 33°C, the situation for the coral control samples had drastically changed from their initial state. The naked control corals died and turned completely white, as it can also be seen in Figure 7. The corals treated with the material without curcumin showed a significant reduction in polyp opening and color change and especially a widespread tissue necrosis. The corals tested in the presence of the material with curcumin showed a very slight morphological and color change compared to the initial condition, as shown in Figure 7. These results demonstrated the effectiveness of the curcumin-containing composite material in counteracting the coral bleaching phenomenon induced by warming.

The composite material, subject of this disclosure, has the advantage of being cheap, easy to produce, totally biodegradable and compatible with aquatic organisms, capable of incorporating and releasing an active agent in a controlled manner.

One or more embodiments of this disclosure may therefore provide a biocompatible and biodegradable composite material for the underwater release of active agents, wherein the composite material comprises at least one hydrophobic compound, at least one hydrophilic compound, at least one active agent.

The at least one hydrophobic compound may be selected from the group consisting of Poly(butyladipate-co-terephthalate) (PBAT), polyhydroxyalkanoates (PHA), zein, silk proteins, albumin, keratin, casein, gelatin, thermoplastic starch.

The at least one hydrophilic compound may be selected from the group consisting of polyvinylpyrrolidone (PVP), polyvinyl alcohol (PVA), polyethylene glycol (PEG), polyethylene oxide (PEO), at least one polysaccharide, preferably selected from alginate, chitosan, pectin, agar, hyaluronic acid.

The at least one active agent may be an antioxidant compound, preferably selected from the group consisting of curcuma, polyphenols, phenolic acids, anthocyanins, flavonoids, beta-carotene, lignin, ascorbic acid, vitamins, butylhydroxyanisole, butylhydroxytoluene.

The hydrophobic compound may be present at an amount equal to or greater than 20% by weight relative to the total weight of the hydrophobic compound and the hydrophilic compound.

The composite material may be in the form of a film, preferably having a thickness ranging from 20 µm to 1.5 cm, preferably from 150 µm to 350 µm.

**In** one or more embodiments, the at least one hydrophobic compound is zein and/or the at least one hydrophilic compound is PVP.

**In** one or more embodiments, the biocompatible and biodegradable composite material comprises zein, **PVP,** curcumin.

The biocompatible and biodegradable composite material may further comprise at least one plasticizer, preferably glycerol and/or at least one emulsifier, preferably soy lecithin, natural gums.

**In** one or more embodiments, the disclosure provides a method for preparing a biocompatible and biodegradable composite material for the underwater release of at least one active agent, which comprises the steps of: - dissolving at least one hydrophilic compound, at least one hydrophobic compound, at least an active agent, and optionally at least one of a plasticizer and an emulsifier, in a solvent to form a solution, - optionally distributing the solution on the surface of a support and evaporating the solvent to obtain the biodegradable composite material in the form of a film.

**In** one or more embodiments, the disclosure provides the use of a biocompatible and biodegradable composite material for the delivery of at least one active agent suitable for preventing and/or treating coral bleaching.

**In** one or more embodiments, the disclosure provides a method for the underwater release of at least one active agent suitable for preventing and/or treating coral bleaching, wherein the method comprises the step of immersing the biocompatible and biodegradable composite material in an aquatic environment. The method may further comprise the step of placing the biocompatible and biodegradable composite material in contact with at least one portion of the surface of at least one coral, preferably wrapping the biodegradable composite material in the form of a film around at least one portion of the coral surface. **In** one or more embodiments, the method may comprise the step of placing the biocompatible and biodegradable composite material in proximity of at least one portion of the surface of at least one coral, in which preferably the biodegradable composite material is in the form of a film and adheres to a support, preferably selected from wires, nettings, tubes, coral nurseries. The disclosure also provides a method for preventing and/or treating coral bleaching, which comprises the step of: - contacting the biodegradable composite material with at least one portion of the surface of at least one coral, preferably wrapping said biodegradable composite material in the form of film around at least one portion of the surface of the coral, and/or - placing the biodegradable composite material in proximity of at least one portion of the surface of at least one coral, wherein preferably said biodegradable composite material is in the form of a film and more preferably adheres to a support, the support being preferably selected from wires, nettings, tubes, coral nurseries.

The disclosure also provides a kit for the production of a biodegradable composite material comprising: - at least one hydrophilic compound, - at least one hydrophobic compound, - optionally at least one active agent, preferably an antioxidant, - optionally at least one plasticizer and/or at least one emulsifier.

## Claims

1. Biocompatible and biodegradable composite material for the underwater release of at least one active agent, wherein the composite material comprises at least one hydrophobic compound, at least one hydrophilic compound, at least one active agent,
wherein the at least one hydrophobic compound is zein, the at least one hydrophilic compound is polyvinylpyrrolidone (PVP), the at least one active agent is curcumin.

2. Biocompatible and biodegradable composite material according to claim 1, wherein the hydrophobic compound is present at an amount equal to or greater than 20% by weight relative to the total weight of the hydrophobic compound and the hydrophilic compound.

3. Biocompatible and biodegradable composite material according to claim 1 or claim 2, wherein said composite material is in the form of a film, preferably having a thickness ranging from 20 µm to 1.5 cm, preferably from 150 µm to 350 µm.

4. Biocompatible and biodegradable composite material according to any one of the preceding claims, further comprising at least one plasticizer, preferably glycerol and/or at least one emulsifier, preferably soy lecithin, natural gums.

5. Method for preparing a biocompatible and biodegradable composite material according to any one of claims 1 to 4 for the underwater release of at least one active agent, wherein the method comprises the steps of:
- dissolving at least one hydrophilic compound, at least one hydrophobic compound, at least one active agent, and optionally at least one of a plasticizer and an emulsifier, in a solvent to form a solution,
- optionally distributing said solution on the surface of a support and evaporating the solvent to obtain the biodegradable composite material in the form of a film.

6. Biocompatible and biodegradable composite material according to any one of claims 1 to 4 for the underwater release of at least one active agent for use in preventing and/or treating coral bleaching.

7. Kit for producing a biocompatible and biodegradable composite material according to any one of claims 1 to 4 comprising:
- at least one hydrophilic compound,
- at least one hydrophobic compound,
- at least one active agent,
- optionally at least one plasticizer and/or at least one emulsifier.

## Patentansprüche

1. Biokompatibles und biologisch abbaubares Verbundmaterial zur Unterwasserfreisetzung mindestens eines Wirkstoffs, wobei das Verbundmaterial mindestens eine hydrophobe Verbindung, mindestens eine hydrophile Verbindung, mindestens einen Wirkstoff umfasst,
wobei die mindestens eine hydrophobe Verbindung Zein ist, die mindestens eine hydrophile Verbindung Polyvinylpyrrolidon (PVP) ist und der mindestens eine Wirkstoff Curcumin ist.

2. Biokompatibles und biologisch abbaubares Verbundmaterial gemäß Anspruch 1, wobei die hydrophobe Verbindung vorhanden ist in einer Menge von mindestens 20 Gew.-% bezogen auf das Gesamtgewicht der hydrophoben Verbindung und der hydrophilen Verbindung.

3. Biokompatibles und biologisch abbaubares Verbundmaterial gemäß Anspruch 1 oder Anspruch 2, wobei das Verbundmaterial in Form eines Films vorliegt, der vorzugsweise eine Dicke im Bereich von 20 µm bis 1,5 cm, vorzugsweise von 150 µm bis 350 µm, aufweist.

4. Biokompatibles und biologisch abbaubares Verbundmaterial gemäß einem der vorstehenden Ansprüche, das ferner mindestens einen Weichmacher, vorzugsweise Glycerin, und/oder mindestens einen Emulgator, vorzugsweise Sojalecithin, natürliche Gummis, umfasst.

5. Verfahren zur Herstellung eines biokompatiblen und biologisch abbaubaren Verbundmaterials gemäß einem der Ansprüche 1 bis 4 zur Unterwasserfreisetzung mindestens eines Wirkstoffs, wobei das Verfahren die folgenden Schritte umfasst:
- Auflösen mindestens einer hydrophilen Verbindung, mindestens einer hydrophoben Verbindung, mindestens eines Wirkstoffs und gegebenenfalls mindestens eines Weichmachers und/oder Emulgators in einem Lösungsmittel zur Bildung einer Lösung,
- gegebenenfalls Verteilen der Lösung auf der Oberfläche eines Trägers und Verdampfen des Lösungsmittels, um das biologisch abbaubare Verbundmaterial in Form eines Films zu erhalten.

6. Biokompatibles und biologisch abbaubares Verbundmaterial gemäß einem der Ansprüche 1 bis 4 zur Unterwasserfreisetzung mindestens eines Wirkstoffs zur Verwendung bei der Verhinderung und/oder Behandlung von Korallenbleiche.

7. Kit zur Herstellung eines biokompatiblen und biologisch abbaubaren Verbundmaterials gemäß einem der Ansprüche 1 bis 4, umfassend:
- mindestens eine hydrophile Verbindung,
- mindestens eine hydrophobe Verbindung,
- mindestens einen Wirkstoff,
- optional mindestens einen Weichmacher und/oder mindestens einen Emulgator.

## Revendications

1. Matériau composite biocompatible et biodégradable pour la libération sous-marine d'au moins un agent actif, lequel matériau composite comprend au moins un composé hydrophobe, au moins un composé hydrophile, au moins un agent actif,
dans lequel l'au moins un composé hydrophobe est la zéine, l'au moins un composé hydrophile est la polyvinylpyrrolidone (PVP), l'au moins un agent actif est la curcumine.

2. Matériau composite biocompatible et biodégradable selon la revendication 1, dans lequel le composé hydrophobe est présent en une quantité égale ou supérieure à 20 % en poids par rapport au poids total du composé hydrophobe et du composé hydrophile.

3. Matériau composite biocompatible et biodégradable selon la revendication 1 ou la revendication 2, lequel matériau composite est sous la forme d'un film, ayant de préférence une épaisseur située dans la plage allant de 20 µm à 1,5 cm, de préférence de 150 µm à 350 µm.

4. Matériau composite biocompatible et biodégradable selon l'une quelconque des revendications précédentes, comprenant en outre au moins un plastifiant, de préférence le glycérol, et/ou au moins un émulsifiant, de préférence la lécithine de soja, les gommes naturelles.

5. Procédé pour préparer un matériau composite biocompatible et biodégradable selon l'une quelconque des revendications 1 à 4 pour la libération sous-marine d'au moins un agent actif, lequel procédé comprend les étapes de :
- dissolution d'au moins un composé hydrophile, d'au moins un composé hydrophobe, d'au moins un agent actif, et éventuellement d'au moins l'un parmi un plastifiant et un émulsifiant, dans un solvant pour former une solution,
- éventuellement distribution de ladite solution sur la surface d'un support et évaporation du solvant pour que soit obtenu le matériau composite biodégradable sous la forme d'un film.

6. Matériau composite biocompatible et biodégradable selon l'une quelconque des revendications 1 à 4 pour la libération sous-marine d'au moins un agent actif destiné à être utilisé dans la prévention et/ou le traitement du blanchiment des coraux.

7. Kit pour produire un matériau composite biocompatible et biodégradable selon l'une quelconque des revendications 1 à 4, comprenant :
- au moins un composé hydrophile,
- au moins un composé hydrophobe,
- au moins un agent actif,
- éventuellement au moins un plastifiant et/ou au moins un émulsifiant.
